(19) Europäisches Patentamt
European Patent Office
Office européen des brevets

(11) **EP 4 285 907 A1**

(12) **EUROPEAN PATENT APPLICATION**
published in accordance with Art. 153(4) EPC


(43) Date of publication:
**06.12.2023 Bulletin 2023/49**

(21) Application number: **22746013.6**

(22) Date of filing: **28.01.2022**

(51) International Patent Classification (IPC):
***A61K 31/5377*** *(2006.01)* ***A61P 35/00*** *(2006.01)*
***A61P 35/04*** *(2006.01)* ***A61P 43/00*** *(2006.01)*

(52) Cooperative Patent Classification (CPC):
**A61K 31/5377; A61P 35/00; A61P 35/04; A61P 43/00**

(86) International application number:
**PCT/JP2022/003228**

(87) International publication number:
**WO 2022/163794 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2021 JP 2021013246**



(71) Applicant: **CHUGAI SEIYAKU KABUSHIKI KAISHA**
**Tokyo 115-8543 (JP)**

(72) Inventors:
- **SATO Jotaro**
**Tokyo 103-8324 (JP)**
- **EMOTO Chie**
**Tokyo 103-8324 (JP)**
- **NAKAMURA Mikiko**
**Tokyo 103-8324 (JP)**

(74) Representative: **Vossius & Partner Patentanwälte Rechtsanwälte mbB**
**Siebertstraße 3**
**81675 München (DE)**


(54) **DRUG COMPOSITION FOR TREATING PEDIATRIC CANCERS**


(57) ALK abnormalities are widely found in a variety of pediatric malignant solid tumors, and ALK inhibitors against ALK abnormalities are considered an important target in the development of treatment in the field of pediatric oncology.

According to the present invention, it is possible to provide a pharmaceutical composition for the treatment of childhood cancer with an ALK abnormality, with a novel dosage and administration, comprising alectinib or a salt thereof, and to be used in the treatment of cancer in children under the age of 2.


[Figure 1]

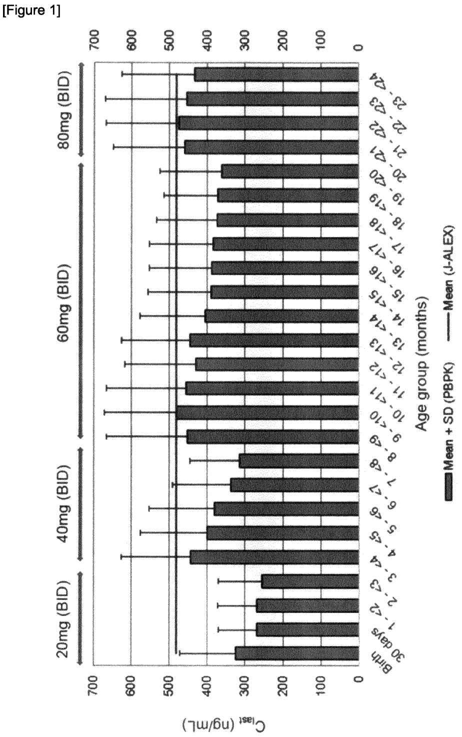


EP 4 285 907 A1

## Description

[Technical Field]

The present invention relates to a pharmaceutical composition for the treatment of cancer in children under the age of 2, which comprises alectinib or a salt thereof, a liquid, a suspension, a use, a method, and the like.

[Background Art]

In recent years, the detection of mutations and amplification of the ALK (Anaplastic Lymphoma Kinase) gene in neuroblastoma has led to demonstrating the existence of "ALK abnormalities" in a plurality of blastomas and sarcomas, including cancers that predominantly occur in children.

The expression of ALK fusion genes was reported relatively early in anaplastic large cell lymphoma (hereinafter, ALCL) and inflammatory myofibroblastic tumor (hereinafter, IMT), which predominantly occur in the pediatric/adolescent and young adult generations. In ALCL, the existence of an NPM-ALK fusion gene by t(2;5)(p23;q35) translocation was first reported in 1994, and then several ALK fusion genes including NPM-ALK have been proven to be expressed in 65-85% of ALCLs (Non Patent Literature 1: Science. 1994 Mar 4; 263(5151): 1281-4., Non Patent Literature 2: Blood. 1997 Mar 1; 89(5): 1483-90.). IMT is a relatively rare soft tissue sarcoma occurring predominantly in the lungs and soft tissues, and occurs predominantly in the teens to twenties. In 2000, when the TPM3/4-ALK fusion gene in IMT was identified, the TPM3/4-ALK fusion gene was then found in approximately 50% of IMTs and ALK translocations were reported to be frequent in pediatric cases (Non Patent Literature 3: Am J Pathol. 2000 Aug; 157(2): 377-84). Recent studies have proven the existence of IMT cases with different combinations of ALK fusions other than the TPK3/4-ALK fusion gene, and have reported that ALK fusion genes are detected in the vast majority of IMTs (Non Patent Literature 4: Cancer Discov. 2014 Aug; 4(8): 889-95).

Amplification or mutation of the ALK gene was also reported in 8 to 14% of neuroblastomas (Non Patent Literature 5: Biochem J. 2008 Dec 1; 416(2): 153-9), and germline mutations of the ALK gene were found to be detected in familial neuroblastoma, which accounts for 1 to 2% of neuroblastomas (Non Patent Literature 6: Nature. 2008 Oct 16; 455(7215): 930-5.). In rhabdomyosarcoma, the amplification of the ALK protein and the presence of an ATIC-ALK fusion gene have also been reported, and although there is variation among the reports, the percentage of "ALK abnormalities" was 18 to 59% (Non Patent Literature 7: Int J Cancer. 2002 Jul 1; 100(1): 49-56; Non Patent Literature 8: Mod Pathol. 2013 Jun; 26(6): 772-81; Non Patent Literature 9: J Clin Oncol. 2012 Jan 20; 30(3): 308-15). Characteristically, the percentage of "ALK abnormalities" has been reported to be high in alveolar rhabdomyosarcoma, a histological type with poor prognosis, and metastatic and recurrent cases have also been reported to have a high percentage of "ALK abnormalities" of around 70% (Non Patent Literatures 8 and 9). Furthermore, it has been reported that the expression of the ALK protein was found in 50% or more of Ewing's sarcomas (Non Patent Literature 10: Int J Cancer. 2013 Jul 15; 133(2): 427-36.), and that in malignant peripheral nerve sheath tumors, malignant fibrous histiocytomas, and some sarcomas such as leiomyosarcoma as well, a certain percentage of the tumors were found to have "ALK abnormalities" (Non Patent Literature 7, and Non Patent Literature 11: Mod Pathol. 2002 Sep; 15(9): 931-8.).

The prognosis for recurrent cases of pediatric malignant solid tumor is poor, with a median overall survival generally ranging from 1 to 2 years regardless of the type of cancer, but there are rare cases of long-time surviving patients for single recurrences or if they are responsive to chemotherapy. Due to the rarity of recurrent pediatric malignant solid tumors, there are very few established standard treatments regardless of the type of cancer, and there is a need for an effective therapeutic drug for childhood cancer regardless of the type of cancer.

Crizotinib, which is a first-generation ALK inhibitor, was clinically developed by the Children's Oncology Group (COG), a U.S. childhood cancer research group, for recurrent/refractory pediatric malignant solid tumors, ALK fusion gene-positive ALCL and neuroblastoma, and other malignant solid tumors including ALK fusion gene-positive IMT, and the efficacy of adult dose-equivalent crizotinib has been reported for pediatric neuroblastoma, IMT and ALCL with ALK gene abnormalities (Non Patent Literature 12: Journal of clinical oncology 2017 Oct 1; 35(28): 3215-21).

[Non Patent Literature 1] Science. 1994 Mar 4; 263(5151): 1281-4.
[Non Patent Literature 2] Blood. 1997 Mar 1; 89(5): 1483-90.
[Non Patent Literature 3] Am J Pathol. 2000 Aug; 157(2): 377-84.
[Non Patent Literature 4] Cancer Discov. 2014 Aug; 4(8): 889-95.
[Non Patent Literature 5] Biochem J. 2008 Dec 1; 416(2): 153-9.
[Non Patent Literature 6] Nature. 2008 Oct 16; 455(7215): 930-5.
[Non Patent Literature 7] Int J Cancer. 2002 Jul 1; 100(1): 49-56.
[Non Patent Literature 8] Mod Pathol. 2013 Jun; 26(6): 772-81.

[Non Patent Literature 9] J Clin Oncol. 2012 Jan 20; 30(3): 308-15.
[Non Patent Literature 10] Int J Cancer. 2013 Jul 15; 133(2): 427-36.
[Non Patent Literature 11] Mod Pathol. 2002 Sep; 15(9): 931-8.
[Non Patent Literature 12] Journal of clinical oncology 2017 Oct 1; 35(28): 3215-21.

[Summary of Invention]

ALK abnormalities are widely found in a variety of pediatric malignant solid tumors, and ALK inhibitors against ALK abnormalities are considered an important target in the development of treatment in the field of pediatric oncology. Particularly, it is desirable to provide a novel therapeutic drug comprising alectinib, a second-generation ALK inhibitor, as an active ingredient, for the treatment of cancer in children under the age of 2. However, there are no pharmacokinetic (PK) data available for alectinib in children under the age of 2, and the developmental changes in the expression of drug-metabolizing enzymes and the like for alectinib need to be considered in addition to the changes in body size associated with growth for children under the age of 2.

Under these circumstances, it is desirable to provide a pharmaceutical composition with a dosage and administration optimized for children under the age of 2.

That is, the present invention provides a pharmaceutical composition, a liquid, a suspension, a use, and a method with the following aspects.

<1> A pharmaceutical composition for the treatment of childhood cancer with an ALK abnormality in children aged 7 months or more and less than 24 months, the composition comprising alectinib or a salt thereof in an amount of 20 mg to 160 mg in terms of free form as a daily dose.

<2> A pharmaceutical composition for the treatment of childhood cancer with an ALK abnormality, the composition comprising:

1) alectinib or a salt thereof in an amount of 20 mg to 120 mg of in terms of free form for children aged 7 months or more and less than 13 months,
2) alectinib or a salt thereof in an amount of 40 mg to 120 mg in terms of free form for children aged 13 months or more and less than 20 months, or
3) alectinib or a salt thereof in an amount of 80 mg to 160 mg in terms of free form for children aged 20 months or more and less than 24 months,
as a daily dose.

<3> A pharmaceutical composition for the treatment of childhood cancer with an ALK abnormality, for administering:

(i) alectinib or a salt thereof at a dose of 20 mg to 60 mg in terms of free form,

1) once or twice daily to children aged 7 months or more and less than 13 months, or
2) twice daily to children aged 13 months or more and less than 20 months, or

(ii) alectinib or a salt thereof at a dose of 40 mg to 80 mg in terms of free form, twice daily to children aged 20 months or more and less than 24 months.

<4> The pharmaceutical composition according to any one of <1> to <3>, wherein the ALK abnormality is an ALK fusion gene, an ALK gene abnormality that is an activating gene mutation or a copy number gain, or the expression of an ALK protein that is abnormal.

<5> The pharmaceutical composition according to any one of <1> to <4>, wherein the childhood cancer is a pediatric malignant solid tumor or a malignant lymphoma.

<5-1> The pharmaceutical composition according to any one of <1> to <4>, wherein the childhood cancer with an ALK abnormality is an ALK-positive pediatric malignant solid tumor or an ALK-positive malignant lymphoma.

<5-2> The pharmaceutical composition according to any one of <1> to <5-1>, wherein the pediatric malignant solid tumor is selected from inflammatory myofibroblastic tumor, sarcoma, central nervous system tumor, cutaneous tumor, retinoblastoma, lung cancer, renal cell carcinoma, anaplastic thyroid carcinoma, thymic carcinoma, ovarian cancer, epithelial tumor of the gallbladder and extrahepatic bile ducts, thyroid cancer, germ cell tumor, malignant fibrous histiocytoma, esophageal cancer, stomach cancer, bowel cancer, breast cancer, and liver cancer.

<5-3> The pharmaceutical composition according to any one of <1> to <5-1>, wherein the malignant lymphoma is Hodgkin's lymphoma or non-Hodgkin's lymphoma.

<5-4> The pharmaceutical composition according to <5-3>, wherein the non-Hodgkin's lymphoma is Burkitt's lymphoma, diffuse large B-cell lymphoma, lymphoblastic lymphoma, or anaplastic large cell lymphoma.
<6> The pharmaceutical composition according to any one of <1> to <5-4>, wherein the childhood cancer is metastatic, curatively unresectable, or has recurrent lesions.
<6-1> The pharmaceutical composition according to any one of <1> to <5-4>, wherein the childhood cancer is refractory or recurrent.
<7> The pharmaceutical composition according to any one of <1> to <6-1>, which is used for oral or tube administration.
<8> A liquid or suspension comprising the pharmaceutical composition according to any one of <1> to <7>.
<9A> A pharmaceutical composition, liquid or suspension comprising alectinib or a salt thereof for administration to children aged 7 months or more and less than 24 months, and having at least one of the characteristics selected from the following (a) to (d), or any combination of two or more thereof:

(a) Dose:

(A) a daily dose of 20 mg to 160 mg in terms of free form;
(B)

1) a daily dose of 20 mg to 120 mg in terms of free form for children aged 7 months or more and less than 13 months,
2) a daily dose of 40 mg to 120 mg in terms of free form for children aged 13 months or more and less than 20 months, or
3) a daily dose of 80 mg to 160 mg in terms of free form for children aged 20 months or more and less than 24 months;

(C)

(i) a dose of 20 mg to 60 mg in terms of free form,

1) once or twice daily to children aged 7 months or more and less than 13 months, or
2) twice daily to children aged 13 months or more and less than 20 months, or

(ii) a dose of 40 mg to 80 mg in terms of free form,
twice daily to children aged 20 months or more and less than 24 months;

(b) Administration method: (A) oral administration; (B) tube administration;
(c) Disease:

(A) child has a childhood cancer (pediatric malignant solid tumor or malignant lymphoma) with an ALK abnormality;
(B) the pediatric malignant solid tumor is selected from inflammatory myofibroblastic tumor, sarcoma, central nervous system tumor, cutaneous tumor, retinoblastoma, lung cancer, renal cell carcinoma, anaplastic thyroid carcinoma, thymic carcinoma, ovarian cancer, epithelial tumor of the gallbladder and extrahepatic bile ducts, thyroid cancer, germ cell tumor, malignant fibrous histiocytoma, esophageal cancer, stomach cancer, bowel cancer, breast cancer, and liver cancer;
(C) the malignant lymphoma is selected from Hodgkin's lymphoma, non-Hodgkin's lymphoma (mature b-cell lymphoma: Burkitt's lymphoma and diffuse large B-cell lymphoma), lymphoblastic lymphoma, and anaplastic large cell lymphoma;

(d) Degree of malignancy:

(A) refractory or recurrent;
(B) metastatic, curatively unresectable;
(C) prior treatment failure.

<9B> A use of alectinib or a salt thereof for producing the pharmaceutical composition, liquid or suspension of <9A>.
<9C> A method for treating childhood cancer with an ALK abnormality, the method comprising administering a pharmaceutical composition comprising alectinib or a salt thereof, having at least one of the characteristics selected from the above (a) to (d), or any combination of two or more thereof.

<9-1> The pharmaceutical composition, liquid, suspension, use and treatment method according to any one of <9A> to <9C>, wherein the ALK abnormality is an ALK fusion gene, an ALK gene abnormality that is an activating gene mutation or a copy number gain, and/or the expression of an ALK protein that is abnormal.

The pharmaceutical composition, liquid, suspension, use, and method of the present invention provide a pharmaceutical composition with a novel dosage and administration, which can be expected to be effective and safe in the treatment of cancer in children under the age of 2.

[Brief Description of Drawings]

[Figure 1] Figure 1 is a graph showing changes in trough concentration when administering 20 mg to 80 mg of alectinib twice daily to a hypothetical Japanese patient aged less than 24 months.
[Figure 2] Figure 2 is a graph showing changes in Cmax and $AUC_{SS}$ for the administration of 20 mg of alectinib once daily to a hypothetical Japanese patient aged less than 13 months.
[Figure 3] Figure 3 is a graph showing changes in trough concentration when administering 20 mg to 40 mg of alectinib twice daily to a hypothetical Japanese patient aged 13 months to less than 24 months.

[Description of Embodiments]

Hereinafter, the definitions of the present invention will be described.
"Alectinib" means the compound represented by formula (I):

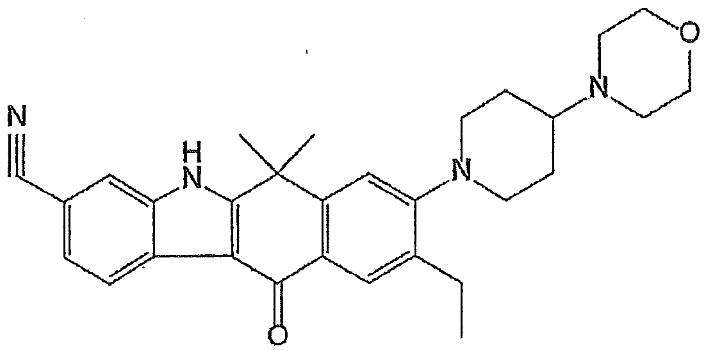

(I)

with the compound name: 9-ethyl-6,6-dimethyl-8-(4-morpholin-4-yl-piperidin-1-yl)-11-oxo-6,11-dihydro-5H-benzo[b]carbazole-3-carbonitrile.
The "salt" of alectinib is preferably a pharmaceutically acceptable salt, and examples of the "pharmaceutically acceptable salt" include hydrochlorides, hydrobromides, hydroiodides, phosphates, phosphonates, sulfates, sulfonates such as methanesulfonate and p-toluenesulfonate, carboxylates such as acetate, citrate, malate, tartrate, succinate, and salicylate, alkali metal salts such as sodium salts and potassium salts; alkaline earth metal salts such as magnesium salts and calcium salts; and ammonium salts such as ammonium salts, alkylammonium salts, dialkylammonium salts, trialkylammonium salts, and tetraalkylammonium salts.
Hydrochlorides are preferred, with monohydrochloride being most preferred.
Alectinib or a salt thereof can be produced by a method known in the art (for example, the methods described in Japanese Patent No. 4588121 and WO2020/050241).
The monohydrochloride of alectinib may be amorphous (WO2016/021707) or crystalline. If crystalline, a crystal having peaks at the diffraction angles (20) near 8.4°, 14.0°, 16.7°, 18.8° and 23.3° in the powder X-ray diffraction pattern is preferred (WO2015/163447). The amorphous form of the monohydrochloride of alectinib can be produced according to the method described in WO2016/021707, and the crystal with these peaks can be produced according to the method described in WO2015/163447.
Alectinib or the salt thereof is contained in an amount of 20 to 70% by weight, preferably 35 to 60% by weight, and still more preferably 45 to 50% by weight, in terms of free form, based on the total amount of the composition.
The composition of the present invention can be formulated using additives such as excipients, lubricants, coating agents, binders, disintegrants, stabilizers, taste and odor improvers, and diluents, and using a well-known method or the methods described in, for example, Japanese Patent No. 4588121, Japanese Patent No. 4918630, Japanese

Patent No. 5006987, Japanese Patent No. 5859712, and WO2020/004630.

Alectinib hydrochloride is a Japanese second-generation ALK inhibitor with high selective inhibitory activity against ALK, developed by Chugai Pharmaceutical Co., Ltd.

Alectinib hydrochloride showed high efficacy in a phase I/II trial (AF-001JP trial) in Japan on patients with ALK fusion gene-positive advanced/recurrent non-small cell lung cancer (hereinafter, NSCLC), and was approved for manufacture and marketing in Japan in July 2014 for "ALK fusion gene-positive unresectable advanced or recurrent non-small cell lung cancer" and in February 2020 for "recurrent or refractory ALK fusion gene-positive anaplastic large cell lymphoma". It is also marketed overseas as a therapeutic drug for "ALK-positive metastatic (advanced) non-small cell lung cancer resistant or intolerant to crizotinib" in numerous countries, and as a therapeutic drug for "ALK-positive advanced non-small cell lung cancer (first-line treatment)" in Europe, the United States, and and the like.

In the present invention, the term "pharmaceutical composition" means a mixture containing at least one active ingredient and at least one inactive ingredient, used for the treatment or prevention of a disease. The term "active ingredient" means an ingredient that exhibits the effect of interest on a living organism, and the term "inactive ingredient" means an ingredient other than an active ingredient, such as an additive. In one aspect of the present invention, the pharmaceutical composition is used in the production of a pharmaceutical preparation. The term "pharmaceutical preparation" means a preparation for the treatment or prevention of a disease.

Pharmaceutical preparations include solid preparations and liquid preparations, but a liquid or suspension is preferred in the present invention. Specific examples of solid preparations include tablets, capsules, liquids, powders, lozenges, chewables, granules, gels, and films, among which granules are preferred.

The pharmaceutical preparation containing the pharmaceutical composition of the present invention is produced by a well-known method using additives such as excipients, lubricants, coating agents, binders, disintegrants, stabilizers, taste and odor improvers, diluents, colorants, fluidizers, preservatives, and antioxidants.

Examples of "excipients" include starches such as corn starch, potato starch, wheat starch, rice starch, partially pregelatinized starch, pregelatinized starch, and porous starch; sugars or sugar alcohols such as lactose hydrate, fructose, glucose, mannitol, and sorbitol; anhydrous dibasic calcium phosphate, crystalline cellulose, precipitated calcium carbonate, and calcium silicate. Examples of preferred excipients include starches such as starch, potato starch, and corn starch, lactose hydrate, crystalline cellulose, and anhydrous dibasic calcium phosphate.

Examples of "coating agents" include ethyl cellulose, hydroxypropyl cellulose, hydroxypropyl methyl cellulose, shellac, talc, carnauba wax, and paraffin.

The "disintegrant" is a component for promoting the rapid disintegration of a solid preparation after its ingestion.

Examples of disintegrants include sodium starch glycolate, low-substituted hydroxypropyl cellulose, calcium carmellose, pregelatinized starch, sodium chloride, corn starch, sodium croscarmellose, crystalline cellulose, anhydrous silicic acid, and carmellose.

The amount of disintegrant used is, for example, 5% or more by weight, preferably 7.5% or more by weight, still more preferably 8.5% or more by weight and particularly preferably 10% or more by weight, based on the whole amount of the composition or preparation of the present invention. The upper limit of the amount used is not particularly limited, but is, for example, 30% by weight. If the preparation of the present invention is a preparation with a coating, such as a coated tablet, the amount used is the amount based on the whole amount of the component coated by the coating (whole amount of the component filled in the capsule, or whole amount of the component coated by the coating).

Examples of "binders" include polyvinylpyrrolidone, macrogol, and the same compounds as the excipients above. Specific examples of the binders include hydroxypropyl cellulose, hydroxypropyl methyl cellulose, methyl cellulose, povidone (polyvinylpyrrolidone), and gum arabic powder. The amount of binder used is preferably 0.1 to 50 parts by weight, and still more preferably 0.5 to 40 parts by weight, based on 100 parts by weight of the preparation.

Suitable examples of "lubricants" include magnesium stearate, calcium stearate, talc, sucrose fatty acid esters, and sodium stearyl fumarate.

Examples of surfactants or emulsifiers include polysorbate 80, polyoxyl 40 stearate, and lauromacrogol.

The colorant may be any colorant as long as it is permitted to be added to medicaments, and examples thereof include food dyes such as food yellow No. 5 (Sunset Yellow, U.S. food yellow No. 6), food red No. 2, and food blue No. 2, lake food colors, and iron sesquioxide.

Examples of stabilizers include p-hydroxybenzoates such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid.

Example of "taste and odor improvers" include commonly used sweeteners, acidulants, and flavoring agents.

The "diluent" is an inactive ingredient added to dilute or the like the active ingredient contained in the preparation. For example, excipients such as lactose or starch, sucrose and the like are used as the inactive ingredient in solid preparations such as granules.

The "fluidizer" is used to improve the fluidity of mixed powders and granules, and typical examples thereof include talc, and light anhydrous silicic acid and hydrated silicon dioxide, which are silicon dioxides. Here, light anhydrous

silicic acid may be any compound composed mainly of hydrated silicon dioxide ($SiO2 \cdot nH2O$) (n indicates an integer), and specific examples thereof include SYLYSIA 320 (trade name, Fuji Silysia Chemical Ltd.) and AEROSIL 200 (trade name, NIPPON AEROSIL Co., Ltd.).

Suitable examples of "preservatives" include p-hydroxybenzoates, chlorobutanol, benzyl alcohol, phenethyl alcohol, dehydroacetic acid, and sorbic acid.

Suitable examples of antioxidants include sulfites and ascorbic acid.

Two or more of the above additives may be used by mixing them in appropriate proportions.

Ethanol, phenol, chlorocresol, purified water, distilled water, and the like can be used as a solvent for producing a liquid.

In the present invention, the term "granules" means a granular material of nearly uniform shape and size obtained by granulating raw materials in powder, lump, solution, or molten liquid form by wet granulation, dry granulation, heated granulation or the like.

The average particle size of the granules is, for example, 150 $\mu$m or more, preferably 180 $\mu$m or more, more preferably 200 $\mu$m or more, still more preferably 250 $\mu$m or more, and particularly preferably 300 $\mu$m or more. The upper limit of the average particle size of the granules is not particularly limited, but is, for example, 1 mm.

The average particle size is obtained by performing the following steps: (i) adding the sampled granulated products on top of a series of sieves with different pore sizes (pore sizes: 850, 500, 355, 250, 180, 106, 75, 53 and 0 $\mu$m), (ii) shaking the sieves for 3 minutes, (iii) measuring the weight of the granulated products remaining on each of the sieves; and (iv) calculating the particle size corresponding to a cumulative percentage of 50% from the pore size and cumulative percentage under the sieve of each sieve using the log-normal distribution approximation. If there are 10% or more by weight of granules having a particle size greater than 850 $\mu$m, the particle size corresponding to a cumulative percentage of 50% is calculated from the pore size and cumulative percentage on the sieve of each sieve using Rosin-Rammler distribution.

The granules used in the present invention preferably have a bulk density of 0.5 g/mL or more and a tap density of 0.6 g/mL or more, and more preferably a bulk density of 0.6 g/mL or more and a tap density of 0.7 g/mL or more. The upper limits for the bulk density and tap density are not particularly limited, but 1.0 g/mL is preferable. Here, the bulk density and tap density are measured according to the method described in the Japanese Pharmacopoeia, 16th Edition.

The pharmaceutical composition used in the present invention may contain a surfactant. Examples of surfactants preferably include monoalkyl sulfates such as sodium lauryl sulfate, sodium tetradecyl sulfate, sodium hexadecyl sulfate, and sodium octadecyl sulfate, dioctyl sodium sulfosuccinate, sodium lauroyl sarcosinate, and sodium dodecylbenzenesulfonate.

In the present invention, "ALK abnormality" is a state presenting with an ALK gene abnormality or the expression of an ALK protein that is abnormal, and "ALK gene abnormality" refers to a state in which activation of ALK is expected due to abnormalities found in the ALK gene, specifically, an ALK fusion gene, an activating gene mutation, or a copy number gain. An "ALK protein that is abnormal" means an ALK protein that is not considered a normal ALK protein, and preferably means a protein expressed by a gene with an ALK gene abnormality. The presence of any ALK abnormality is also referred to as ALK-positive.

1. ALK fusion gene

ALK, a receptor-type tyrosine kinase, is activated by dimerization via ligand binding, but when this gene is translocated, it is thought to be activated by permanent dimerization via the coiled-coil domain bound to ALK without ligand binding. In an ALK fusion gene, chromosomal inversion or translocation of a gene encoding a protein having the capability to multimerize with the ALK gene leads to the formation of a fusion gene of the two, which results in the expression of an ALK fusion protein that is a fusion of the two. The ALK fusion protein permanently activates the ALK tyrosine kinase, which results in a continuous aberrant activation of the cell proliferation signals, and thereby causes the canceration of cells. EML4, NPM, TPM3, TFG, ATIC, CLTC1, MSN, TPM4, ALO17, MYH9, RANBP2, CARS, SEC31L1, and the like are known as partner genes that form ALK fusion genes ("Guidance for ALK Gene Testing," Biomarker Committee, the Japan Lung Cancer Society).

ALK fusion genes can be detected by fluorescence in situ hybridization (FISH), immunohistochemistry (IHC), RT-PCR (including genome sequencing), and gene panel testing (hybrid-capture method and amplicon method), and includes known ALK fusion genes as well as unknown ALK fusion genes detected by these methods.

As a kit for detecting ALK fusion genes or proteins expressed by ALK fusion genes, the Vysis LSI ALK Break Apart Rearrangement Probe Kit (registered trademark), Vysis ALK Break Apart FISH Probe Kit (registered trademark) (Abbott), Histofine ALK iAEP (registered trademark) kit (Nichirei), Ventana OptiView ALK (D5F3) (Roche Diagnostics K.K.), FoundationOne (registered trademark) CDx Cancer Genomic Profile (Chugai Pharmaceutical Co., Ltd.), OncoGuide AmoyDx (registered trademark) ROS1 Fusion Gene Detection kit (RIKEN GENESIS Co., Ltd.), and Oncomine Dx Target Test Multi-CDx System (Life Technologies Japan Ltd.) can be used. If an ALK fusion gene or ALK fusion protein

is detected by these kits, it is referred to as ALK fusion gene-positive.

2. ALK activating gene mutation

An ALK activating gene mutation means that the ALK gene has been mutated to an active form by point or frameshift mutations. Specifically, G1 128A, F1 174L, F1174I, F1174S, R1192Q, F1245C, F1245L, Y1278S, M1166R, I1171N, R1275Q, and the like are known, but those newly detected using WGS or targeted capture sequencing are also included. G1128A, F1174L, F1174I, F1174S, R1192Q, F1245C, F1245L, Y1278S, M1166R, R1275Q are preferred. WGS (whole genome sequencing) is the most comprehensive technique for decoding all the genome regions using next-generation sequencers. Not only single-nucleotide variants (SNV), but also insertions or deletions (INDEL), copy-number variations (CNV, including loss, gain, loss of heterozygosity, etc.), large scale structural variations (SV, including translocations, inversions, deletions, duplications, etc.), exogenous genomes (such as EBV and HTLV-1 genomes), and the like can be detected. It is particularly effective in detecting genetic and structural abnormalities in non-coding regions. Targeted capture sequencing is a method for detecting genetic abnormalities commonly used in "cancer gene panel testing".

3. Copy number gain

The term "copy number gain" refers to the presence of 2.5 or more copies (or a Log2 ratio $\geq$ 0.32) of a particular gene in a target diploid genome. If the copy number is 2.5, 50% of the cells used to define the copy number contain two copies of the gene as usual in diploid organisms and 50% of the cells used to define the copy number contain the usual two copies and one more copy of the gene (three copies of the gene in total). The copy number gain can be analyzed using MLPA and CGH.
MLPA (Multiplex Ligation-dependent Probe Amplification) is a method for quantitatively analyzing of copy number variation in a target genomic region by amplifying probes hybridized to template DNA by PCR.
CGH (comparative genomic hybridization)/array CGH/SNPs array are methods for comprehensively analyzing genomic copy number variations. Genomic DNA extracted from tumor tissues and control tissues is labeled with different fluorescent dyes and then mixed and hybridized to metaphase chromosomes. The genomic copy number variation in tumor tissue is detected by observing the ratio of fluorescent signals.

The term "childhood cancer" generally means a cancer occurring in children aged 15 years or less. Childhood cancers have different properties from adult cancers, with about half classified as hematologic tumors such as leukemia and lymphoma, 15% as brain tumors, and most pediatric solid tumors other than brain tumors as sarcomas, which occur deep in the body, or embryonal tumors, which originate from undifferentiated cells before their functions are yet determined in their respective body locations
(https://www.ncc.go.jp/jp/rcc/about/paediatric_malignancies/index.html). Many ALK abnormalities due to ALK gene abnormalities or amplification/high expression of ALK are found in childhood cancers.

Examples of childhood cancers in the present invention include pediatric malignant solid tumors and malignant lymphomas.

Examples of pediatric malignant solid tumors include inflammatory myofibroblastic tumor, rhabdomyosarcoma, Ewing sarcoma family of tumors (Ewing sarcoma of the bone and other tissues, primitive neuroectodermal tumor (PNET), Askin's tumor (PNET occurring primarily in the chest wall), etc.), sarcomas such as leiomyosarcoma, central nervous system tumors such as neuroblastoma, glioma, neuroblastoma, and malignant peripheral nerve sheath tumor, cutaneous tumors such as malignant melanomas and Spitz tumors, retinoblastoma, lung cancers such as non-small cell lung cancer, renal cell carcinoma, anaplastic thyroid carcinoma, thymic carcinoma, and ovarian cancer, as well as epithelial tumor of the gallbladder and extrahepatic bile ducts, thyroid cancer, germ cell tumor, and malignant fibrous histiocytoma.

Malignant melanomas include: (1) acral lentiginous melanoma (ALM), (2) superficial spreading melanoma (SSM), (3) nodular melanoma (NM), and (4) lentigo maligna melanoma (LMM).

Spitz tumors include malignant Spitz tumors, juvenile malignant melanoma, Spitzoid melanoma, and Spitz nevus-like melanoma.

Pediatric malignant solid tumors are known to be rare cancers, with rare cancer being a generic term for malignant tumors (cancers) that occur less frequently. In Japan, the annual incidence is less than 6 cancer cases per 100,000 persons.

In addition to the above solid cancers, rare histological subtypes of non-rare cancers are also included.

In the present invention, rare histological subtypes of non-rare cancers are rare histological subtypes of esophagus cancer, stomach cancer, bowel cancer, breast cancer, liver cancer, and the like, and are specifically the cancer types classified in Table 1 below. In addition, the rare histological subtypes of cancer that are not classified in Table 1 below and are reported to have an annual incidence of less than 6 cases per 100,000 persons (for example, fibrolamellar carcinoma, a particular form of hepatocellular carcinoma) are also included in the rare histological subtypes of non-rare

cancers in the present invention.

[Table 1]

| | |
|---|---|
| Epithelial tumors of the stomach | |
| | Squamous cell carcinoma with variants of stomach |
| | Salivary gland-type tumors of stomach |
| | Undifferentiated carcinoma of stomach |
| Epithelial tumors of the colon | |
| | Squamous cell carcinoma with variants of colon |
| Epithelial tumors of the liver and intrahepatic bile ducts | |
| | Hepatocellular carcinoma of liver and intrahepatic bile ducts |
| | Cholangiocarcinoma of IBT |
| | Adenocarcinoma with variants of liver and IBT |
| | Undifferentiated carcinoma of liver and IBT |
| | Squamous cell carcinoma with variants of liver and IBT |
| | Bile duct cystadenocarcinoma of IBT |
| Epithelial tumors of the breast | |
| | Invasive lobular carcinoma of breast |
| | Mammary Paget's disease of breast |
| | Special types of adenocarcinoma of breast |
| | Metaplastic carcinoma of breast |
| | Salivary gland-type tumors of breast |
| | Epithelial tumor of male breast |

Malignant lymphoma is a rare childhood cancer and is classified into Hodgkin's lymphoma and non-Hodgkin's lymphoma. Childhood non-Hodgkin's lymphoma is further classified into mature b-cell lymphoma (Burkitt's lymphoma and diffuse large B-cell lymphoma), lymphoblastic lymphoma, and anaplastic large cell lymphoma, and is preferably anaplastic large cell lymphoma or diffuse large B-cell lymphoma.

Examples of the childhood cancer in the present invention preferably include inflammatory myofibroblastic tumor, neuroblastoma, thymic carcinoma, ovarian cancer, anaplastic thyroid carcinoma, neuroblastoma, Spitz tumor, malignant melanoma, rhabdomyosarcoma, Ewing's sarcoma, retinoblastoma, and glioma of the central nervous system.

In another aspect of the present invention, inflammatory myofibroblastic tumor, diffuse large B-cell lymphoma, and neuroblastoma are preferred as the childhood cancer.

Yet another aspect of the present invention is a pharmaceutical composition for the treatment of childhood cancers such as thymic carcinoma, ovarian cancer, Spitz tumor, malignant melanoma, retinoblastoma, diffuse large B-cell lymphoma, or rare histological subtypes of stomach cancer, bowel cancer, breast cancer, or liver cancer, which have an ALK abnormality.

The above childhood cancers with refractory or recurrent lesions are preferred.

The term "recurrent" means that the optimal response to the most recent treatment was a complete or partial remission, and the term "refractory" means that the best response to the most recent treatment was stable or advanced. Pathological conditions such as metastatic, curatively unresectable are also included.

"Prior treatment failure" means a case in which the prior treatment was ineffective and discontinued. Examples of prior treatments include chemotherapy with other agents having similar or different mechanisms of action, radiation therapy, and surgery. Specific examples include cases in which resistance is developed by the administration of crizotinib.

There has been several reports that crizotinib, an ALK inhibitor, is effective in recurrent/refractory childhood cancers. Case reports of crizotinib on IMT with an ALK fusion gene (The New England journal of medicine. 2010 Oct 28; 363(18): 1727-33.), a US COG phase I trial in children with solid tumors or ALCL (The Lancet Oncology. 2013 May; 14(6): 472-80.), and a phase II trial evaluating the efficacy of crizotinib in ALK fusion gene-positive ALCL and neuroblastoma, and other malignant solid tumors including ALK fusion gene positive IMT (Journal of clinical oncology 2017 Oct 1; 35(28): 3215-21.) have shown that crizotinib has clinical efficacy in these childhood cancers.

Results from a phase III trial in Japanese adults with ALK fusion gene-positive NSCLC showed that alectinib had better outcomes than crizotinib in terms of both safety and efficacy. In addition, alectinib has been used since 2014 and its safety is better established due to its extensive experience in use in Japanese adults with NSCLC.

These findings allow to predict the efficacy of alectinib in childhood cancers with an ALK abnormality.

The term "treatment" means an act of administering the pharmaceutical composition of the present invention to a subject for the purpose of causing either the death of the cancer cells of a childhood cancer or a decrease in the number of those cells, suppressing the growth of cancer cells, and improving the various symptoms caused by the cancer.

Specifically, it refers to any of the following cases.

- Tumor size has not increased compared to pre-treatment, and has preferably decreased compared to pre-treatment
- Progression-free survival is prolonged compared to the no-treatment group or the conventional treatment group
- Overall survival is prolonged compared to the no-treatment group or the conventional treatment group
- The disease control rate is 10% or more, 20% or more, 30% or more, or 40% or more, or greater than that of the no-treatment group or the conventional treatment group.

The disease control rate is defined as the percentage of patients whose best overall response is either CR, PR, or SD, based on an overall evaluation determined by the combination of response of target lesions, response of non-target lesions, and appearance of new lesions, as will be described in Example 2.

It also includes slowing the progression of a childhood cancer compared to pre-treatment, the no-treatment group or the conventional treatment group, and improving general condition and clinical parameters compared to pre-treatment.

The size of the tumor is measured by imaging tests using CT (computed tomography) and MRI (magnetic resonance imaging). Tumor size is determined by the sum of tumor diameters. As the tumor diameter, the longest is measured for non-nodal lesions and the shortest for nodal lesions. Tumor diameter is measured by CT or MRI cross-sectional imaging.

"Progression-free survival (PFS)" refers to the time from treatment (or randomization) to first disease progression or death. In one aspect of the present invention, PFS can be assessed by the Response Evaluation Criteria in Solid Tumors (RECIST). In one aspect of the present invention, PFS can be assessed by CA-125 levels as a determinant of progression.

The pharmaceutical composition of the present invention comprises alectinib or a salt thereof in an amount of 20 mg to 160 mg in terms of free form as a daily dose, according to age in month, for childhood cancer patients aged 7 months or more and less than 24 months. The above dose is equivalent to a daily dose of 2 mg to 16 mg in terms of a free form of alectinib or a salt thereof per kg of the male and female average body weight by age in month based on the "Technical report for Japanese National Growth Survey for infants and children in 2010" by the Ministry of Health, Labour and Welfare, for childhood cancer patients aged 7 months or more and less than 24 months.

For children aged 7 months or more and less than 13 months, alectinib or a salt thereof may be administered at a daily dose of 20 mg to 120 mg in terms of free form. More preferably, it may be administered at a daily dose of 20 mg, 40 mg, 80 mg, or 120 mg. Alternatively, a single dose of 20 mg may be administered once or twice daily, a single dose of 40 mg twice daily, or a single dose of 60 mg twice daily.

For children aged 13 months or more and less than 20 months, alectinib or a salt thereof in terms of free form may be administered at a daily dose of 40 mg to 120 mg in terms of free form. More preferably, it may be administered at a daily dose of 40 mg, 80 mg, or 120 mg. Alternatively, a single dose of 20 mg may be administered twice daily, a single dose of 40 mg twice daily, or a single dose of 60 mg twice daily.

For children aged 20 months or more and less than 24 months, alectinib or a salt thereof in terms of free form may be administered at a daily dose of 80 mg to 160 mg in terms of free form. More preferably, it may be administered at daily doses of 80 mg, 120 mg, or 160 mg. Alternatively, a single dose of 40 mg may be administered twice daily, a single dose of 60 mg twice daily, or a single dose of 80 mg twice daily.

Alectinib is known to be metabolized by the metabolic enzyme CYP3A4, but the existence of other metabolic pathways has also been suggested. The activity and expression levels of metabolic enzymes differ between children and adults, and it is not clear how they change according to developmental stage in children. Therefore, the dosage and administration for children was established based on PBPK simulation assuming that a developmental process similar to that of CYP3A4 exists in the developmental profile of an unspecified metabolic enzyme in children, the lower limit was established at a dosage and administration that lowers the risk of the estimated child exposure exceeding adult exposure as much as possible, and the upper limit was established based on PBPK simulation assuming the same metabolic activity as adults from 0 month of age.

Specifically, the dosage and administration is as described in the following (A) to (P).

(A) A pharmaceutical composition for the treatment of childhood cancer with an ALK abnormality, the composition comprising:

1) alectinib or a salt thereof in an amount of 20 mg in terms of free form (a single dose of 20 mg once daily) for children aged 7 months or more and less than 13 months,
2) alectinib or a salt thereof in an amount of 40 mg in terms of free form (a single dose of 20 mg twice daily) for children aged 13 months or more and less than 20 months, or
3) alectinib or a salt thereof in an amount of 80 mg in terms of free form (a single dose of 40 mg twice daily) for children aged 20 months or more and less than 24 months,

as a daily dose.
(B) A pharmaceutical composition for the treatment of childhood cancer with an ALK abnormality, the composition comprising:

1) alectinib or a salt thereof in an amount of 20 mg in terms of free form (a single dose of 20 mg once daily) for children aged 7 months or more and less than 13 months,
2) alectinib or a salt thereof in an amount of 80 mg in terms of free form (a single dose of 40 mg twice daily) for children aged 13 months or more and less than 20 months, or
3) alectinib or a salt thereof in an amount of 80 mg in terms of free form (a single dose of 40 mg twice daily) for children aged 20 months or more and less than 24 months,

as a daily dose.
(C) A pharmaceutical composition for the treatment of childhood cancer with an ALK abnormality, the composition comprising:

1) alectinib or a salt thereof in an amount of 20 mg of in terms of free form (a single dose of 20 mg once daily) for children aged 7 months or more and less than 13 months,
2) alectinib or a salt thereof in an amount of 80 mg in terms of free form (a single dose of 40 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(D) A pharmaceutical composition for the treatment of childhood cancer with an ALK abnormality, the composition comprising:

1) alectinib or a salt thereof in an amount of 20 mg in terms of free form (a single dose of 20 mg once daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(E) A pharmaceutical composition for the treatment of childhood cancer with an ALK abnormality, the composition comprising:

1) alectinib or a salt thereof in an amount of 20 mg in terms of free form (a single dose of 20 mg once daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 160 mg in terms of free form (a single dose of 80 mg twice daily) for children aged 20 months or more and less than 24 months,

as a daily dose.
(F) A pharmaceutical composition for the treatment of childhood cancer with an ALK abnormality, the composition comprising:

1) alectinib or a salt thereof in an amount of 40 mg in terms of free form (a single dose of 20 mg twice daily) for children aged 7 months or more and less than 13 months,
2) alectinib or a salt thereof in an amount of 40 mg in terms of free form (a single dose of 20 mg twice daily) for children aged 13 months or more and less than 20 months, or
3) alectinib or a salt thereof in an amount of 80 mg in terms of free form (a single dose of 40 mg twice daily) for children aged 20 months or more and less than 24 months,

as a daily dose.
(G) A pharmaceutical composition for the treatment of childhood cancer with an ALK abnormality, the composition comprising:

1) alectinib or a salt thereof in an amount of 40 mg in terms of free form (a single dose of 20 mg twice daily) for children aged 7 months or more and less than 13 months,
2) alectinib or a salt thereof in an amount of 80 mg in terms of free form (a single dose of 40 mg twice daily) for children aged 13 months or more and less than 20 months, or
3) alectinib or a salt thereof in an amount of 80 mg in terms of free form (a single dose of 40 mg twice daily) for children aged 20 months or more and less than 24 months,

as a daily dose.
(H) A pharmaceutical composition for the treatment of childhood cancer with an ALK abnormality, the composition comprising:

1) alectinib or a salt thereof in an amount of 40 mg in terms of free form (a single dose of 20 mg twice daily) for children aged 7 months or more and less than 13 months,
2) alectinib or a salt thereof in an amount of 80 mg in terms of free form (a single dose of 40 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(I) A pharmaceutical composition for the treatment of childhood cancer with an ALK abnormality, the composition comprising:

1) alectinib or a salt thereof in an amount of 40 mg in terms of free form (a single dose of 20 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(J) A pharmaceutical composition for the treatment of childhood cancer with an ALK abnormality, the composition comprising:

1) alectinib or a salt thereof in an amount of 40 mg in terms of free form (a single dose of 20 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 160 mg in terms of free form (a single dose of 80 mg twice daily) for children aged 20 months or more and less than 24 months,

as a daily dose.
(K) A pharmaceutical composition for the treatment of childhood cancer with an ALK abnormality, the composition comprising:

1) alectinib or a salt thereof in an amount of 80 mg in terms of free form (a single dose of 40 mg twice daily) for children aged 7 months or more and less than 13 months,
2) alectinib or a salt thereof in an amount of 80 mg in terms of free form (a single dose of 40 mg twice daily) for

children aged 13 months or more and less than 20 months, or
3) alectinib or a salt thereof in an amount of 80 mg in terms of free form (a single dose of 40 mg twice daily) for children aged 20 months or more and less than 24 months,

as a daily dose.
(L) A pharmaceutical composition for the treatment of childhood cancer with an ALK abnormality, the composition comprising:

1) alectinib or a salt thereof in an amount of 80 mg in terms of free form (a single dose of 40 mg twice daily) for children aged 7 months or more and less than 13 months,
2) alectinib or a salt thereof in an amount of 80 mg in terms of free form (a single dose of 40 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(M) A pharmaceutical composition for the treatment of childhood cancer with an ALK abnormality, the composition comprising:

1) alectinib or a salt thereof in an amount of 80 mg in terms of free form (a single dose of 40 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(N) A pharmaceutical composition for the treatment of childhood cancer with an ALK abnormality, the composition comprising:

1) alectinib or a salt thereof in an amount of 80 mg in terms of free form (a single dose of 40 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 160 mg in terms of free form (a single dose of 80 mg twice daily) for children aged 20 months or more and less than 24 months,

as a daily dose.
(O) A pharmaceutical composition for the treatment of childhood cancer with an ALK abnormality, the composition comprising:

1) alectinib or a salt thereof in an amount of 120 mg in terms of free form (a single dose of 60 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 120 mg in terms of free form (a single dose of 60 mg twice daily) for children aged 20 months or more and less than 24 months,

as a daily dose.
(P) A pharmaceutical composition for the treatment of childhood cancer with an ALK abnormality, the composition comprising:

1) alectinib or a salt thereof in an amount of 120 mg in terms of free form (a single dose of 60 mg twice daily) for children aged 7 months or more and less than 13 months,
2) alectinib or a salt thereof in an amount of 120 mg in terms of free form (a single dose of 60 mg twice daily) for children aged 13 months or more and less than 20 months, or
3) alectinib or a salt thereof in an amount of 160 mg in terms of free form (a single dose of 80 mg twice daily) for children aged 20 months or more and less than 24 months,

as a daily dose.

The pharmaceutical composition of the present invention can be administered orally or by tube. The pharmaceutical composition of the present invention can also be administered as a liquid or suspension by dissolving or disintegrating it in a liquid, such as water or a beverage, at the time of use. Specifically, a capsule may be administered orally using an oral jelly. Alternatively, a capsule may be administered orally after disintegrating it with water or the like to form a suspension. Tube administration is a method for administering a liquid using a tube inserted through the nose or mouth to the stomach (nasogastric tube or orogastric tube). Specifically, the capsule may be made into a suspension using warm water, water, or the like, and the suspension may be administered by tube.
The pharmaceutical composition of the present invention may be taken before, between, or after meals.
When administered once a day, it is preferably administered in the morning, and when administered twice a day, in the morning and at night.
The pharmaceutical composition of the present invention may be used in combination with the following drugs and therapeutic interventions such as supportive care (including transfusion therapy) and symptomatic treatment, if deemed clinically necessary.

1) Antiemetic, antidiarrheal, antipyretic
2) Treatment and operative therapy as clinically necessary concomitant therapies (e.g., fractures from car accidents)
3) Prophylactic or therapeutic administration of antimicrobial agents
4) Bisphosphonate preparations and anti-RANKL antibodies for bone lesions
5) Administration of anticonvulsants
Anticonvulsants may be administered in patients with intracranial tumors.
6) Administration of intracranial antihypertensive drugs and steroids
Intracranial antihypertensive drugs (such as a formulation of concentrated glycerin, mannitol, and isobide) and steroids (such as dexamethasone (Decadron Injection)) may be administered in patients with intracranial tumors.

[Examples]

Hereinafter, the present invention will be specifically described with reference to the descriptions of Examples, but the present invention is not to be construed as limited by these descriptions.
All patents and references expressly cited herein are incorporated herein by reference in their entirety.

Example 1

The maximum dosage and administration at which the average exposure at steady state is not exceeded when repeatedly administering 300 mg of the present drug twice daily (BID) to adults in the J-ALEX study (Japanese Phase III clinical trial, JO28928) was examined as a dosage and administration of alectinib for children. The physiologically-based pharmacokinetic (PBPK) model was used to predict the exposure when alectinib is orally administered to the children.
Alectinib is primarily metabolized by CYP3A4. For pediatric PK prediction, especially for children under the age of 2 years, the need to consider developmental changes in the expression of drug-metabolizing enzymes and the like, in addition to the changes in body size associated with growth, has been reported (C Wagner, P Zhao, Y Pan, V Hsu, J Grillo, S M Huang, V Sinha. Application of Physiologically Based Pharmacokinetic (PBPK) Modeling to Support Dose Selection: Report of an FDA Public Workshop on PBPK. CPT Pharmacometrics Syst Pharmacol. 2015 Apr; 4(4): 226-30.). Therefore, for PK prediction of alectinib in children up to about 2 years of age, an analysis taking into consideration the developmental changes in the expression of metabolic enzymes is particularly important. Thus, a PBPK model incorporating changes in age-dependent physiological parameters, including changes in body size associated with age and developmental changes in CYP3A4 expression, was constructed to predict the pediatric PK of alectinib.
The PBPK model was constructed by combining alectinib-specific physicochemical information (molecular weight, logP, pKa, etc.), and the in vitro and in vivo study results obtained so far, with the physiological information on the target population in the SimCYP (registered trademark) software (Certara, Sheffield, UK) (Table 2).

[Table 2]

| Input parameters of alectinib for PBPK modelling using SimCYP(R) population-based ADME simulator (Version 19) | |
|---|---|
| Parameters | Values |
| *Physicochemical* | |
| Mol weight (g/mol) | 482.6 |
| log P | 1.96 |
| Compound type | Monoprotic Base |
| pKa 1 | 7.05 |
| B/P (blood-to-plasma ratio) | 2.64 |
| Free fraction in plasma | 0.003 |
| *Absorption* | |
| Absorption model | First-order (assumed) |
| $fu_{Gut}$ | 0.01 |
| $Q_{GUT}$ (L/h) | 8.49 |
| Fa[A)] | 0.48 |
| ka (1/h)[A)] | 0.22 |
| Lag time (h)[A)] | 2.92 |
| *Distribution* | |
| Volume of distribution method | Full PBPK with Method 2 (Rodgers method) |
| Predicted $V_{ss}$ (L/kg) [B)] | 6.31 |
| Actual $V_{ss}$ (Ukg) [c)] | 6.35 |
| *Elimination* | |
| $CL_{int}$, CYP3A4 | 9.98 μL/min/pmol of isoform |
| $CL_{int}$, CYP2J2[D)] | 285 μL/min/pmol of isoform |
| CLint, $HLM_{other}$ | 1710 μL/min/mg protein |
| *Drug-drug interaction* | |
| Competitive CYP2C8 inhibition $K_I$ (μM) | 0.147 |
| Mechanism-based Inhibitor CYP3A4 inhibition $K_I$ (μM) | 8.29 |
| Mechanism-based inhibitor CYP3A4 $k_{inact}$ (1/h) | 3.74 |
| CYP3A4 induction $Ind_{stope}$ (1/μM) | 3.52 |

A PBPK model of alectinib was optimized based on the developed model.
A) Used for pediatric PBPK simulations. Absorption parameters was estimated by fitting clinical PK profiles observed in Japanese adult patients (JP28927: multiple p.o. administration with a standard meal, where formulation of alectinib was a 150mg capsule).
B) Adjusted by Kp scalar- 0.26.
C) V. (475 L) after Intravenous administration of alectinib was reported by Morcos et al. To calculate a body-weight normalized value, 74.8kg was used. This value was reported as the average value of the target subjects.
D) CYP2J2 was arbitrarily assigned although the true enzyme responsible is not known.
*: Morcos PN, Yu L, Bogman K, Sato M, Katsuki H, Kawashima K, et al. Absorption, distribution, metabolism and excretion (ADME) of the ALK inhibitor alectinib: results from an absolute bioavailability and mass balance study in healthy subjects. Xenobiotica. 2017 Mar; 47(3): 217-29.

This model was initially validated using clinical PK results in healthy adults (Table 3). It was then optimized

using clinical trial results in Japanese adults as parameters related to the absorption process were incorporated into the model.

[Table 3]

| Summary of clinical studies used in the PBPK modeling | | | | | | |
|---|---|---|---|---|---|---|
| Study/Phase | Subject | Fedl Fasted | N | Formulation | Treatment | PK assay site |
| NP28989/Phase 1 Period 1 (Absolute BA) Xenobiotica. 2017 Mar; 47(3):217-229. 1 | Healthy volunteer | Fed | 6 | 150 mg capsule | po: 600 mg single dose iv: 50 pg of [$^{14}C$]-alectinib containing ~18.5 kBq (500 nCi) of $^{14}C$ radiolabeled material | Q Squared Solutions |
| NP28989/Phase Period 2 (ADME) Xenobiotica. 2017 Mar;47(3): 217-229. | Healthy volunteer | Fed | 6 | Suspension | 600 mg single dose of [$^{14}C$]-alectinib containing ~2.5 MBq (67 μCi) of $^{14}C$ radiolabeled material | Q Squared Solutions |
| JP28927/Phase 1 Period 3 (Food effect) Cancer Sci. 2016 Nov; 107(11): 1642-1646 | ALK+NSCLC adult patient | Fed | 3 | 150 mg capsule | 300 mg BID | Chugai |

Based on the parameters in Table 2, the following dosages and administrations were estimated through PBPK simulation, assuming the same metabolic activity as adults from 0 months of age for an elimination process of unknown mechanism.

[Table 4]

| Expected dosage and administration for children aged less than 24 months (Upper Limit) | |
|---|---|
| Age Group | Dosage and Administration |
| Less than 3 months | 20 mg, BID |
| 3 months or more and less than 7 months | 40 mg, BID |
| 7 months or more and less than 13 months | 60 mg, BID |
| 13 months or more and less than 20 months | 60 mg, BID |
| 20 months or more and less than 24 months | 80 mg, BID |

The following BID doses were estimated not to exceed the adult exposure: 20 mg, BID for children aged less than 3 months; 40 mg, BID for children aged 3 months or more and less than 7 months; 60 mg, BID for children aged 7 months or more and less than 20 months; and 80 mg, BID for children aged 20 months or more and less than 24 months (Figure 1). That is, the dosages and administrations listed in Table 4 are the maximum dosages and administrations for children under 24 months of age.

To predict pediatric PK, the physiological information on Japanese adults was replaced with that on Japanese children. This physiological information on Japanese children is contained in the SimCYP (registered trademark) Pediatric module and includes parameters to take account of the age-dependent physiological and anatomical features of neonates (full term birth), infants and children (e.g. age-associated changes in organ size and blood flow, developmental changes in the expression of metabolic enzymes, renal function, and blood protein concentration, etc.). Then, for the PK prediction in children under the age of 2 years, the developmental changes in the enzyme expression involved in the elimination of alectinib were assumed. CYP3A4, the major metabolic enzyme of alectinib, is considered to contribute to about 40% of hepatic metabolic clearance; however, the metabolic enzymes responsible for the remaining 60% of clearance have not yet been identified. The CYP3A4 ontogeny model available in the SimCYP (registered trademark) Pediatric module and the slow ontogeny model for the elimination process of unknown mechanism were used for the developmental changes in metabolic enzymes. The pediatric developmental profile of unidentified metabolic enzymes was assumed to have a developmental process similar to that of CYP3A4, to lower the risk of the estimated child exposure exceeding

adult exposure as much as possible. The inter-individual variability in blood concentration on this simulation was calculated based on Japanese demographic data (Japanese pediatric demographic data source from Japanese Ministry of Health, Labour, and Welfare. Available from: https://www.mhlw.go.jp/content/000451760.pdf (Access as of April 16, 2020)), in addition to the variation in each physiological parameter.

The pediatric dosage and administration was examined as the maximum dosage and administration at which the estimated exposure in children does not exceed the average exposure at steady state when repeatedly administering 300 mg of the present drug BID to adults in the J-ALEX study (Japanese Phase III clinical trial, JO28928, Lancet Volume 390, Issue 10089, 1-7 July 2017, Pages 29-39). The preparation used in the study is in capsule form, but the capsule is in principle administered by tube as a solution suspended in water since it is assumed to be difficult for children under the age of 2 years to swallow it. Since the gastrointestinal absorption profile when administering a suspension and the solubility of alectinib in the pediatric gastrointestinal tract are unknown, absorption process in children was assumed to be the same as in adults, and the absorption parameters, Fa, ka, and lag times estimated from the plasma alectinib concentration in Japanese adults (JP28927, Period 3), were used.

$C_{trough}$ (trough value), which were observed in the J-ALEX study, was used as the index of exposure at BID administration. However, since the administration in adults is BID, if changed to QD (once daily) at the same dosage, the $C_{max}$ (maximum blood concentration) or $AUC_{ss}$ (area under the steady-state blood concentration-time curve) may exceed the J-ALEX study even if $C_{trough}$ is less than equivalent. Therefore, in the study of QD, the dosages at which $C_{max}$ and $AUC_{ss}$ do not exceed the exposure in the J-ALEX study were examined. As an index of the adult exposure, $C_{max}$ and $AUC_{ss}$ were calculated with the population pharmacokinetic (PopPK) model for alectinib developed using PK data in the J-ALEX study.

The following dosages and administrations were estimated through PBPK simulation, assuming a slow ontogeny for an elimination process of unknown mechanism:

In children under 7 months of age, the average estimated exposure ($C_{max}$ and $AUC_{ss}$) after multiple QD administrations of 20 mg exceeded the adult exposure (Figure 2), suggesting that the estimated regimen is difficult to apply to this age group.

For children aged 7 months or more and less than 13 months, a QD administration of 20 mg was estimated not to exceed the adult exposure (Figure 3).

For children aged 13 months or more and less than 20 months, a BID administration of 20 mg was estimated not to exceed the adult exposure ($C_{trough}$) (Figure 3).

For children aged 20 months or more and less than 24 months, a BID administration of 40 mg was estimated not to exceed the adult exposure ($C_{trough}$) (Figure 3).

The above results are summarized in Table 5.

[Table 5]

| Estimated dosage and administration for children aged less than 24 months (Lower Limit) | |
|---|---|
| Age Group | Dosage and Administration |
| 7 months or more and less than 13 months | 20 mg, QD |
| 13 months or more and less than 20 months | 20 mg, BID |
| 20 months or more and less than 24 months | 40 mg, BID |

These results showed that in children under 7 months of age, the average estimated exposure for a multiple QD administrations of 20 mg exceeded the adult exposure, suggesting that the estimated regimen is difficult to apply to this age group. For children aged 7 months or more, a dosage and administration not exceeding the adult exposure was estimated as shown in Table 5.

Therefore, it is considered that at least this dosage and administration can ensure efficacy and safety in cancers of children aged 7 months or more and less than 2 years with an ALK abnormality.

The results of Tables 4 and 5 are summarized in Table 6.

[Table 6]

| Expected dosage and administration for children aged less than 24 months | |
|---|---|
| Age Group | Dosage and Administration |
| Less than 3 months | 20 mg, BID or less |
| 3 months or more and less than 7 months | 40 mg, BID or less |

(continued)

| Expected dosage and administration for children aged less than 24 months | |
|---|---|
| Age Group | Dosage and Administration |
| 7 months or more and less than 13 months | 20 mg, QD to 60 mg, BID |
| 13 months or more and less than 20 months | 20 mg, BID to 60 mg, BID |
| 20 months or more and less than 24 months | 40 mg, BID to 80 mg, BID |

Therefore, it was found that the efficacy and safety in cancers of children under the age of 2 with an ALK abnormality are obtained within the dosages and administrations listed in Table 6.

Example 2: Clinical trial

The efficacy and safety of alectinib in cancers of children aged 7 months or more and less than 2 years with an ALK abnormality is confirmed in the clinical trial described below.

1. Test Method

Childhood cancer patients aged 7 months or more and less than 2 years, who were confirmed to have an ALK abnormality by screening prior to the start of the trial, are administered alectinib according to Table 7, "Dose of alectinib per month of age in pediatric patients under the age of 2," or 2.2 below, from the age in month at trial enrollment.

20 mg capsules of alectinib are used in the present trial. Since the pediatric patients in the present trial often have difficulty taking capsules, oral jelly may be used, or a simple suspension method, in which the capsules are suspended in warm water to make a solution, can be used to administer orally or by tube.

Pediatric patients aged 7 months or more and less than 2 years are administered alectinib using the starting dosage until three courses are completed, even if their age changes or they pass the age of 2. From the fourth course until the patient turns 2 years old, administration is continued by changing the dosage according to Table 7, "Dose of alectinib per month of age in pediatric patients under the age of 2" according to the age in month at the start of each course. Once over 2 years old, the dose of alectinib is determined using the "Dose of alectinib per body weight in pediatric patients aged 2 years or more and 15 years or less" at the start of the next course. From then on, administration of alectinib is continued without dosage modification, even if there is weight fluctuation during the period of the trial.

Administration is preferably spaced at 8 hour intervals.

One course consists of 28 days, and treatment is continued unless the criteria for discontinuation of protocol treatment are met.

In addition, in principle, oral administration will be continued unless the criteria for course interruption are met, regardless of the time of evaluation within each course.

[Table 7]

Dose of alectinib per month of age in pediatric patients under the age of 2

| | **Daily dose** | **Single dose (morning I evening)** |
|---|---|---|
| 7 months or more and less than 13 months | 20 mg | 20 mg 10 mg |
| 13 months or more and less than 20 months | 40 mg | 20 mg / 20 mg |
| 20 months or more and less than 24 months | 80 mg | 40 mg / 40 mg |

2.1 Special (simple suspension) cohort

A special cohort of 1-6 young pediatric patients who are unable to take capsules orally ("simple suspension cohort") is established and administered alectinib by a simple suspension. Details of the suspension method and oral administration method are described in the "Procedures for simple suspension cohort". A single dose of alectinib is administered according to Table 7.

2.2 Single dose (Course 0: C0)

In the simple suspension cohort, or in children having a body weight of less than 15 kg and from whom PK blood samples for pharmacokinetic evaluation can be collected, a single dose is administered as a course 0 only for the first dose for pharmacokinetic evaluation after a single dose. In course 0, the investigational drug is taken once in the morning on day 1 (C0day1) only. The evening dose is not taken, and then administration is stopped for 2 days. For the other patients, administration of alectinib is started from course 1, without single dose administration (course 0).

3. Endpoints during protocol treatment

Each endpoint was evaluated according to the schedule in Table 8 below.

[Table 8]

| Study calendar 2: For special cohort, or children having body weight of less than 15 kg and from whom PK blood samples for pharmacokinetic evaluation can be collected | | | | | | | | | | | | | |
|---|---|---|---|---|---|---|---|---|---|---|---|---|---|
| Endpoint | Screening | Top: course, bottom; day | | | | | | | | | | Treatment discontinuation *7 *15 or observation | Follow-up *15 |
| | | 0 | | | 1 | | | | 2 | 3 | 4 and after | | |
| | | day 1 | day 2 | day 3 | day 1 *17 | day 8 | day 15 | day 28 | day 1 | day 1 | day 1 | | |
| Consent | ■ *1 | | | | | | | | | | | | |
| Subject background | ■ *2 | | | | | | | | | | | | |
| ECOG-PS *25 | ■ *3 | ■ *4 | | | ■ | ■ | | | ■ | ■ | ■ | ■ | |
| Height | ■ *3 | | | | | | | | | | | | |
| Body weight | ■ *3 | | | | ○ | | | | | | | | |
| Vital signs | ■ *3 | ■ *4 | | | ■ *4 | ■ | | | ■ | ■ | ■ | ■ | |
| Hematological test | ■ *3 | | | | | ■ | | | ■ | ■ | ■ *14 | ■ | |
| Biochemical test | ■ *3 | | | | | ■ | | | ■ | ■ | ■ *14 | ■ | |
| Test for infectious diseases | ■ *2 | | | | | | | | | | | | |
| Urine test | ■ *3 | | | | | | | | | | | | |
| Pregnancy test | ■ *3 | | | | | | | | | | | | |
| 12-lead Electrocardiogram | ■ *3 | | | | | | | | | ■ | ■ *5 | ■ | |
| Chest X-ray | ■ *3 | | | | | ■ | | | ■ | ■ | ■ *5 | ■ | |
| Tumor assessment (CT/MRI) | ■ *3 | | | | | | | | | | | ■ *6 | |
| Adverse events | | ■ | | | ■ *4 | ■ | | | ■ | ■ | ■ | ■ *8 | |

(continued)

| Study calendar 2: For special cohort, or children having body weight of less than 15 kg and from whom PK blood samples for pharmacokinetic evaluation can be collected | | | | | | | | | | | | | |
|---|---|---|---|---|---|---|---|---|---|---|---|---|---|
| Endpoint | Screening | Top: course, bottom; day | | | | | | | | | | Treatment discontinuation *7 *15 or observation | Follow-up *15 |
| | | 0 | | | 1 | | | | 2 | 3 | 4 and after | | |
| | | day 1 | day 2 | day 3 | day 1 *17 | day 8 | day 15 | day 28 | day 1 | day 1 | day 1 | | |
| Concomitant therapy | ■ *3 | ■ | | | ■ | ■ | | | ■ | ■ | ■ | ■ | |
| Survival confirmation, post-treatment survey | | | | | | | | | | | | | ■ |
| PK sample for pharmacokinetic evaluation | | ■ *18 | ■ *18 | ■ *18 | ■ *19 | ■ *20 | ■ *20 | ■ *21 | ■ *22 | | ■ *23 | | |
| FoundationOne test | ■ | | | | | | | | | | | | |
| Blood sample for NGS *16 *24 | ■ *9 (20 mL) | | | | | | | | | | | | |
| VENTANAALK(D5F3) CDx Assay *16 | ■ *12 | 1 | | | | | | | | | | | |
| Blood sample for ancillary study*16*24 | ■ *9 (10 mL) | | | | | | | | | ■ *10 (20 mL) | ■ *10 (20 mL) | ■ *11(20 mL) | |
| Tumor tissue for ancillary study*16 | O *12 | | | | | | | | | O *13 | | ■ *13 | |

4. Evaluation of efficacy

Imaging tests (CT or MRI) for tumor assessment are performed every 8 weeks until 24 weeks and every 12 weeks after 25 weeks, without change once the treatment protocol has started, even if there was a delay in starting the course. The same modality and imaging conditions used in the pre-enrollment assessment are used*. Imaging tests are allowed within ±7 days of the above stipulated date.

If brain metastases are found by a screening test, the same modality as at screening (contrast-enhanced CT or contrast-enhanced MRI*) is continued for subsequent tumor assessments.

If CR or PR is observed for the first time as overall response, imaging tests for confirmation are performed 4 weeks or more later.

*If contrast medium allergy or renal function deterioration newly occurs, the assessment is continued by simple CT/MRI.

5. Measurement of blood concentration of alectinib

Measurement of the blood concentration of alectinib is performed in the special cohort, or in patients having a body weight of less than 15 kg and from whom PK blood samples for pharmacokinetic evaluation can be collected.

Point and volume of blood collection

The volume of a single blood collection for measuring the blood concentration of alectinib is 2 mL, and the points of blood collection are shown in the table below (however, for a body weight of less than 10 kg, the volume of a single blood collection is 1.5 mL).

In the case of a twice-daily administration, "administration" in Table 9 refers to the first oral administration of the day.

[Table 9]

| **Point of blood collection** | | | **Acceptable range (min)** | **Volume of blood collection (mL/ collection)** | **Remarks** |
|---|---|---|---|---|---|
| C0 | day 1 | Before administration | | 2 [5)] | Course 0 Total blood collection |
| | | 1 hour after administration | ± 15 min | 2 [5)] | |
| | | 4 hours after administration | ± 30 min | 2 [5)] | Volume: 14 mL[6)] |
| | | 6 hours after administration | ± 30 min | 2 [5)] | |
| | | 10 hours after administration | ± 30 min | 2 [5)] | |
| | day 2 | 24 hours after administration | ± 120 min | 2 [5)] | |
| | day 3 | 48 hours after administration | ± 120 min | 2 [5)] | |

(continued)

| Point of blood collection | | | Acceptable range (min) | Volume of blood collection (mL/ collection) | Remarks |
|---|---|---|---|---|---|
| C1 | day 1 | Before administration | ± 120 min 1) | 2 5) | Course 1 Total blood collection volume: 16 mL7) |
| | day 8 | Before administration | ± 120 min 4) | 2 5) | |
| | day 15 | Before administration | ± 120 min 4) | 2 5) | |
| | day 28 | Before administration | ± 120 min 4) | 2 5) | |
| | | 1 hour after administration | ± 15 min | 2 5) | |
| | | 4 hours after administration | ± 30 min | 2 5) | |
| | | 6 hours after administration | ± 30 min | 2 5) | |
| | | 10 hours after administration 2) | ± 30 min | 2 5) | |
| C2 | day 1 | Before administration 3) | ± 120 min 4) | 2 5) | |
| C4 | day 1 | Before administration | ± 120 min 4) | 2 5) | |

1) Should be measured within the acceptable range of ± 120 minutes, starting at 72 hours after administration on day 1 of C0.
2) Should be measured before the second dose for cases taking alectinib twice daily.
3) Performed only in patients taking alectinib once daily.
4) Should be measured within the acceptable range of ± 120 minutes, starting at 24 hours after the morning dose on the previous day.
5) The volume of a single blood collection should be 1.5 mL for patients having a body weight of less than 10 kg.
6) The total volume of blood collection for course 0 should be 10.5 mL for a body weight of less than 10 kg.
7) The total volume of blood collection for course 1 should be 12 mL for a body weight of less than 10 kg.

[Notes]

Referring to the standard for blood collection in sick children (21), the volume of blood collection should not exceed 3 mL/kg per day and 10% of total body blood volume (8 mL/kg) at 8 weeks.

6. Response evaluation

The tumor shrinkage response was evaluated according to the following procedure in accordance with the "New response evaluation criteria in solid tumours: Revised RECIST guideline (version 1.1), Japanese translation version by JCOG."

6.1 Baseline evaluation

The neoplastic lesions prior to enrollment are identified by contrast-enhanced CT of the head, chest, abdomen, and pelvis (slice thickness: 5 mm or less), and each lesion is classified as a "measurable lesion" or "non-measurable lesion".

(1) Definition of measurable lesion

1) A lesion that meets any of the following is considered as a measurable lesion.

(i) lesion other than a nodal lesion (non-nodal lesion), with a maximum diameter of 10 mm or more on CT or MRI
(ii) nodal lesion with a shortest diameter of 15 mm or more on CT (nodal lesions with a shortest diameter of 10 mm or more and less than 15 mm are considered as non-target lesions, and the lymph nodes with a shortest diameter of less than 10 mm are not considered as lesions)

2) All lesions other than those listed above are considered as non-measurable lesions.

(2) Selection of target lesions

Of the measurable lesions found at enrollment, up to 5 in order of increasing diameter (longest diameter for non-nodal lesions and shortest diameter for nodal lesions) and up to 2 per organ* are selected as target lesions.

* How "organs" are counted

- Left and right organs (such as the lungs and kidneys) are considered together as one organ.
- All lymph nodes are considered as one organ, regardless of the site

3) Baseline documentation of non-target lesions

All lesions not selected as target lesions, whether measurable or not, are recorded as non-target lesions. Multiple non-target lesions in the same organ are recorded as one lesion (e.g., multiple enlarged pelvic lymph nodes, multiple liver metastases).

6.2 Evaluation of tumor shrinkage response

Target and non-target lesions are evaluated at 8, 16, and 24 weeks, and every 12 weeks thereafter according to "4. Evaluation of efficacy" using the same testing method as at enrollment, regardless of any delay in starting the course, and the diameter of the target lesions and disappearance or progression of non-target lesions is recorded.

(1) Response evaluation criteria

1) Response evaluation criteria for target lesions

(i) CR (Complete Response)
If all non-nodal target lesions have disappeared and all nodal target lesions have a shortest diameter of less than 10 mm.
(ii) PR (Partial Response)
30% or more decrease in the sum of diameters of target lesions, compared to the baseline sum of diameters
(iii) PD (Progressive Disease)
20% or more increase in the sum of diameters of target lesions and a 5 mm or more absolute increase in the sum of diameters, compared to the smallest sum of diameters during the trial (if the baseline is the smallest during the trial, this is considered as the smallest sum of diameters)
(iv) SD (Stable Disease)
Neither shrinkage corresponding to PR nor increase corresponding to PD
(v) NE (Not Evaluable)
If testing cannot be performed for any reason, or if CR, PR, PD, or SD cannot be determined

The rate of reduction or increase in the sum of diameters is calculated as follows.

[Expression 1]

$$\text{Rate of reduction in sum of diameters} = \frac{\text{Sum of diameters pre-treatment - Sum of diameters at evaluation}}{\text{Sum of diameters pre-treatment}} \times 100\%$$

$$\text{Rate of increase in sum of diameters} = \frac{\text{Sum of diameters at evaluation - Smallest sum of diameters}}{\text{Smallest sum of diameters}} \times 100\%$$

(i) The actual measurements of the diameter of target lesions should be recorded as long as they are measurable (for example, even when less than 5 mm), but if the diameter of the target lesion is considered to be "too small to measure," the diameter should be recorded as 0 mm when no tumor lesion is considered to be persisting and as 5 mm when a tumor lesion is considered to be persisting, regardless of the CT slice thickness.
(ii) It should be recorded as PD if the rate of reduction meets the conditions for PR and at the same time the rate of increase meets the conditions for PD.
(iii) If a lesion has split during treatment, each diameter should be added to the sum of diameters.
(iv) If multiple lesions coalesce during treatment and the boundaries can no longer be distinguished, the diameter of the coalesced lesions should be added to the sum of diameters. If the lesions are adjacent to each other but the boundaries of the lesions can be distinguished, the diameter of each lesion should be added to the sum of diameters.

2) Response evaluation criteria for non-target lesions

(i) CR (Complete Response)
If all non-nodal non-target lesions have disappeared and all nodal non-target lesions have a shortest diameter of less than 10 mm
(ii) Non-CR/non-PD
If one or more non-target lesions persists (including the persistence of nodal non-target lesions with a shortest diameter of 10 mm or more)
(iii) PD (Progressive Disease)
"Unequivocal progression" of existing non-target lesions (including recurrence).
(iv) NE (Not Evaluated)
If testing could not be performed for any reason, or if CR, non-CR/non-PD, or PD cannot be determined

3) Appearance of new lesions

If a lesion that was not present at baseline is found after the start of treatment, it is considered that a "new lesion" has appeared.

(2) Overall Response

Overall response is determined from the combination of response of target lesions, response of non-target lesions, and appearance of new lesions according to Table 10 at 8, 16, and 24 weeks, and every 12 weeks thereafter. In the absence of non-target lesions at baseline, the overall response is determined by the response of target lesions and the appearance of new lesions.

[Table 10]

| Target lesion | Non-target lesion | New lesion | Overall response |
|---|---|---|---|
| CR | CR | No | CR |

(continued)

| Target lesion | Non-target lesion | New lesion | Overall response |
|---|---|---|---|
| CR | Non-CR / non-PD | No | PR |
| CR | NE | No | PR |
| PR | Non-PD or NE | No | PR |
| SD | Non-PD or NE | No | SD |
| NE | Non-PD | No | NE |
| PD | Any | Yes or No | PD |
| Any | PD | Yes or No | PD |
| Any | Any | Yes | PD |

(3) Best Overall Response

The overall response is considered as "good" in the order of CR > PR > SD > PD > NE, and the Best Overall Response is determined according to the following criteria. If the definition of several categories is met, it should be classified into the better one in the order of CR > PR > SD > PD > NE.

(i) CR (Complete Response)
If CR is obtained as overall response for two or more times in a row at intervals of 4 weeks (28 days) or more.
(ii) PR (Partial Response)
If an overall response of PR or better (CR or PR) is obtained for two or more times in a row at intervals of 4 weeks (28 days) or more.
(iii) SD (Stable Disease)
If a best overall response of neither CR nor PR is obtained and the overall response is SD or better at least once.
(iv) PD (Progressive Disease)
If overall response is PD, without meeting a best overall response of any of CR, PR, or SD
(v) NE (Not Evaluable)
If all overall responses are NE

[Industrial Applicability]

The pharmaceutical composition of the present invention is particularly useful as a therapeutic drug for cancer in children aged less than 24 months.

## Claims

1. A pharmaceutical composition for the treatment of childhood cancer with an ALK abnormality in children aged 7 months or more and less than 24 months, the composition comprising alectinib or a salt thereof in an amount of 20 mg to 160 mg in terms of free form as a daily dose.

2. A pharmaceutical composition for the treatment of childhood cancer with an ALK abnormality, the composition comprising:

    1) alectinib or a salt thereof in an amount of 20 mg to 120 mg in terms of free form for children aged 7 months or more and less than 13 months,
    2) alectinib or a salt thereof in an amount of 40 mg to 120 mg in terms of free form for children aged 13 months or more and less than 20 months, or
    3) alectinib or a salt thereof in an amount of 80 mg to 160 mg in terms of free form for children aged 20 months or more and less than 24 month,

    as a daily dose.

3. A pharmaceutical composition for the treatment of childhood cancer with an ALK abnormality, for administering:

(1) alectinib or a salt thereof at a dose of 20 mg to 60 mg in terms of free form,

1) once or twice daily to children aged 7 months or more and less than 13 months, or
2) twice daily to children aged 13 months or more and less than 20 months, or

(2) alectinib or a salt thereof at a dose of 40 mg to 80 mg of in terms of free form, twice daily to children aged 20 months or more and less than 24 months.

4. The pharmaceutical composition according to any one of claims 1 to 3, wherein the ALK abnormality is an ALK fusion gene, an ALK gene abnormality that is an activating gene mutation or a copy number gain, or the expression of an ALK protein that is abnormal.

5. The pharmaceutical composition according to any one of claims 1 to 4, wherein the childhood cancer is a pediatric malignant solid tumor or a malignant lymphoma.

6. The pharmaceutical composition according to any one of claims 1 to 4, wherein the childhood cancer is metastatic, curatively unresectable, or has recurrent lesions.

7. The pharmaceutical composition according to any one of claims 1 to 6, which is used for oral or tube administration.

[Figure 1]

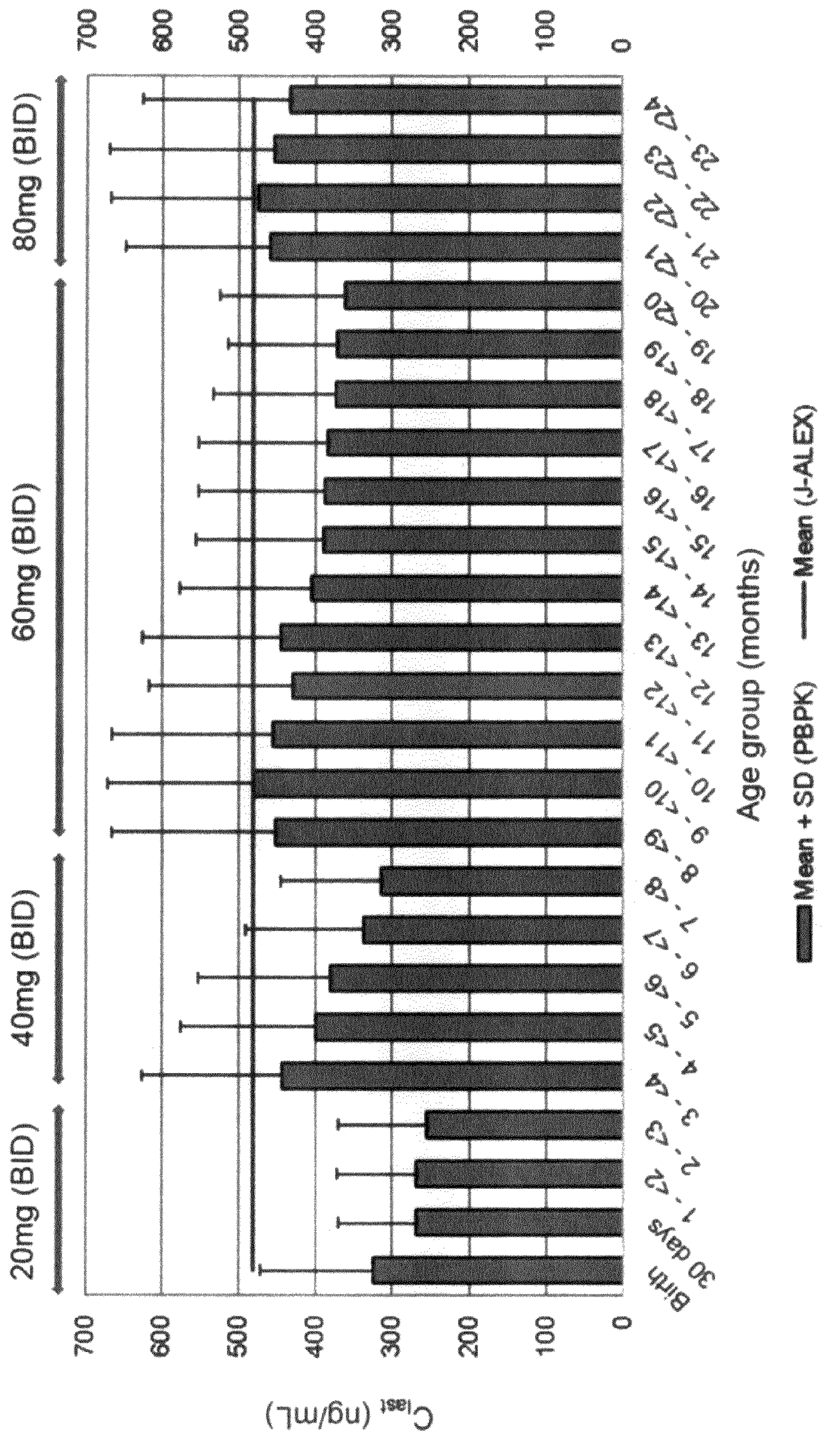
20mg (BID)
40mg (BID)
60mg (BID)
80mg (BID)
$C_{last}$ (ng/mL)
0
100
200
300
400
500
600
700
Birth - 30 days
1 - <2
2 - <3
3 - <4
4 - <5
5 - <6
6 - <7
7 - <8
8 - <9
9 - <10
10 - <11
11 - <12
12 - <13
13 - <14
14 - <15
15 - <16
16 - <17
17 - <18
18 - <19
19 - <20
20 - <21
21 - <22
22 - <23
23 - <24
Age group (months)
Mean + SD (PBPK)
Mean (J-ALEX)

[Figure 2]

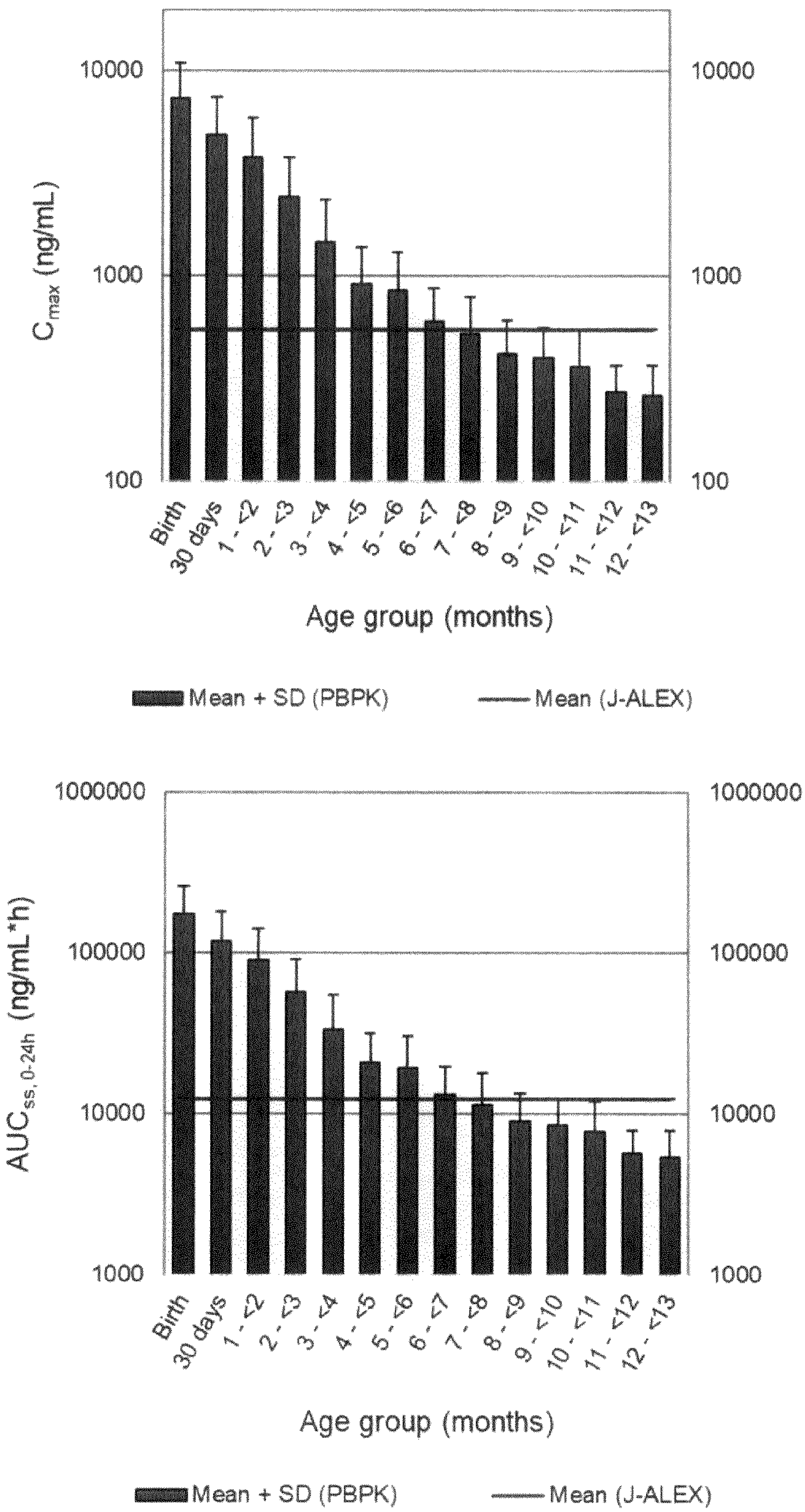
10000
1000
100
$C_{max}$ (ng/mL)
Birth
30 days
1 - <2
2 - <3
3 - <4
4 - <5
5 - <6
6 - <7
7 - <8
8 - <9
9 - <10
10 - <11
11 - <12
12 - <13
Age group (months)
Mean + SD (PBPK)
Mean (J-ALEX)
1000000
100000
10000
1000
$AUC_{ss, 0-24h}$ (ng/mL*h)
Age group (months)
Mean + SD (PBPK)
Mean (J-ALEX)

[Figure 3]

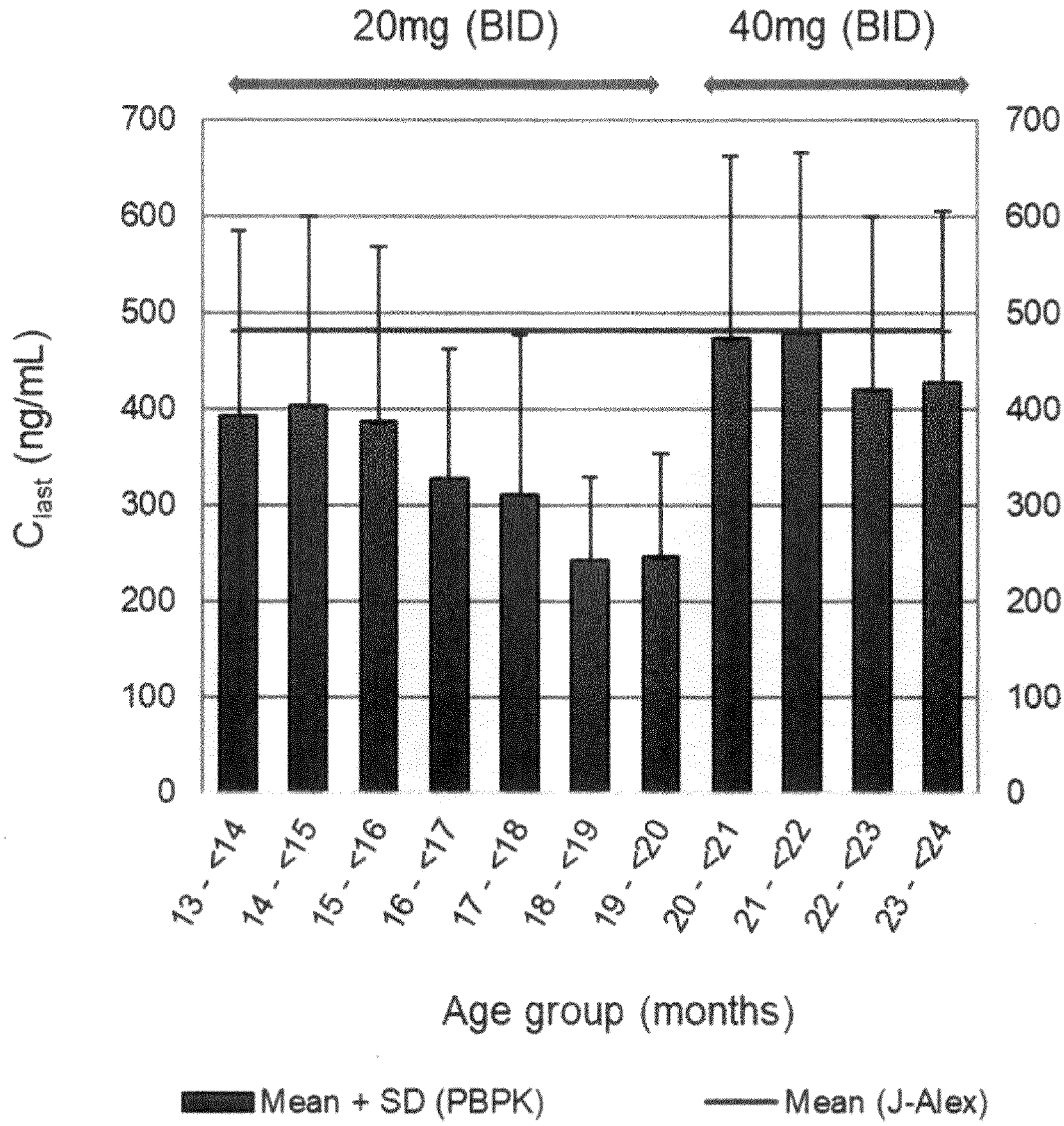
20mg (BID)
40mg (BID)
700
600
500
400
300
200
100
0
Clast (ng/mL)
13 - <14
14 - <15
15 - <16
16 - <17
17 - <18
18 - <19
19 - <20
20 - <21
21 - <22
22 - <23
23 - <24
Age group (months)
Mean + SD (PBPK)
Mean (J-Alex)

**INTERNATIONAL SEARCH REPORT**

International application No.
**PCT/JP2022/003228**

**A. CLASSIFICATION OF SUBJECT MATTER**

***A61K 31/5377***(2006.01)i; ***A61P 35/00***(2006.01)i; ***A61P 35/04***(2006.01)i; ***A61P 43/00***(2006.01)i
FI: A61K31/5377; A61P35/00; A61P35/04; A61P43/00 111

According to International Patent Classification (IPC) or to both national classification and IPC

**B. FIELDS SEARCHED**

Minimum documentation searched (classification system followed by classification symbols)

A61K31/00-33/44

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | NUNEZ, O. L. et al. Infantile inflammatory myofibroblastic tumors: clinicopathological and molecular characterization of 12 cases. Modern Pathology. 2020, vol. 33, pages 576-590<br>page 578, right column, second paragraph, table 1, page 579, table 2, page 583, second paragraph, page 587, right column, second paragraph | 1-7 |
| Y | CORTE, C. M. D. et al. Role and targeting of anaplastic lymphoma kinase in cancer. Molecular Cancer. 2018, vol. 17, no. 30, pages 1-9<br>page 5, left column, second paragraph to right column, second paragraph | 1-7 |
| A | 公益社団法人日本医師会 治験促進センター, 難治性悪性固形腫瘍または悪性リンパ腫の小児患者を対象としたアレクチニブの医師主導第I相試験, 臨床試験登録システム, 15 June 2018, obtained from the Internet (URL: https://dbcentre3.jmacct.med.or.jp/jmactr/App/JMACTRE02_04_org/JMACTRE02_04_org.aspx?kbn=3&seqno=7897) (obtained on 15 February 2022), (CENTER FOR CLINICAL TRIALS, JAPAN MEDICAL ASSOCIATION.), non-official translation (Alectinib Physician-Initiated Phase I, Clinical Trial Registration System for Pediatric Patients with Refractory Malignant Solid Tumors or Malignant Lymphoma.)<br>entire text, 特に"●選択/除外基準"の"年齢下限", non-official translation (particularly, the "lower limit of age" in "Selection/Exclusion Criteria") | 1-7 |

☑ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 February 2022** | **01 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/003228**

**C. DOCUMENTS CONSIDERED TO BE RELEVANT**

| Category* | Citation of document, with indication, where appropriate, of the relevant passages | Relevant to claim No. |
|---|---|---|
| P, X | 公益社団法人日本医師会 治験促進センター, ALK遺伝子異常を有する希少がんに対するアレクチニブの医師主導治験, 臨床試験登録システム, 01 February 2021, obtained from the Internet (URL: https://dbcentre3.jmacct.med.or.jp/JMACTR/App/JMACTRE02_04/JMACTRE02_04.aspx?kbn=3&seqno=8259) (obtained on 15 February 2022), (CENTER FOR CLINICAL TRIALS, JAPAN MEDICAL ASSOCIATION.), non-official translation (Alectinib's physician-initiated clinical trial and clinical trial registration system for rare cancers with ALK gene abnormalities.)<br>entire text (特に"●介入の内容"の"投与経路/適用部位"及び"投与回数/使用回数", "●選択/除外基準"の"年齢下限"及び"選択基準"), non-official translation (particularly, "Route of administration/Site of application" and "Number of administrations/Number of uses" in "Contents of intervention", "Lower age limit" and "Selection criteria" in "Selection/exclusion criteria") | 1-7 |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4588121 B
- WO 2020050241 A
- WO 2016021707 A
- WO 2015163447 A
- JP 4918630 B
- JP 5006987 B
- JP 5859712 B
- WO 2020004630 A
- JP 28927 A


### Non-patent literature cited in the description


- *Science,* 04 March 1994, vol. 263 (5151), 1281-4
- *Blood,* 01 March 1997, vol. 89 (5), 1483-90
- *Am J Pathol.,* August 2000, vol. 157 (2), 377-84
- *Cancer Discov.,* August 2014, vol. 4 (8), 889-95
- *Biochem J.,* 01 December 2008, vol. 416 (2), 153-9
- *Nature,* 16 October 2008, vol. 455 (7215), 930-5
- *Int J Cancer.,* 01 July 2002, vol. 100 (1), 49-56
- *Mod Pathol.,* June 2013, vol. 26 (6), 772-81
- *J Clin Oncol.,* 20 January 2012, vol. 30 (3), 308-15
- *Int J Cancer.,* 15 July 2013, vol. 133 (2), 427-36
- *Mod Pathol.,* September 2002, vol. 15 (9), 931-8
- *Journal of clinical oncology,* 01 October 2017, vol. 35 (28), 3215-21
- *The New England journal of medicine.,* 28 October 2010, vol. 363 (18), 1727-33
- *The Lancet Oncology,* May 2013, vol. 14 (6), 472-80
- Technical report for Japanese National Growth Survey for infants and children in 2010. Ministry of Health, Labour and Welfare
- **C WAGNER ; P ZHAO ; Y PAN ; V HSU ; J GRILLO ; S M HUANG ; V SINHA.** Application of Physiologically Based Pharmacokinetic (PBPK) Modeling to Support Dose Selection: Report of an FDA Public Workshop on PBPK. *CPT Pharmacometrics Syst Pharmacol,* April 2015, vol. 4 (4), 226-30
- **MORCOS PN ; YU L ; BOGMAN K ; SATO M ; KATSUKI H ; KAWASHIMA K et al.** Absorption, distribution, metabolism and excretion (ADME) of the ALK inhibitor alectinib: results from an absolute bioavailability and mass balance study in healthy subjects. *Xenobiotica,* March 2017, vol. 47 (3), 217-29
- *Xenobiotica,* March 2017, vol. 47 (3), 217-229
- *Cancer Sci.,* November 2016, vol. 107 (11), 1642-1646
- Japanese pediatric demographic data source. Japanese Ministry of Health, Labour, and Welfare, 16 April 2020
- Japanese Phase III clinical trial, JO28928. *Lancet,* 01 July 2017, vol. 390 (10089), 29-39